# EUROPEAN PATENT SPECIFICATION

(11) **EP 2 746 250 B1**
(45) Date of publication and mention of the grant of the patent: **27.09.2017**
(21) Application number: 12199109.5
(22) Date of filing: 21.12.2012
(51) Int. Cl.: C07C 227/16, C07C 229/36, C07B 59/00

(54) **Precursors and process for the production of 18F-labelled amino acids**
Vorläufer und Verfahren für die Herstellung von 18F-markierten Aminosäuren
Précurseurs et procédé pour la production d'acides aminés marqués 18F

(43) Date of publication of application: 25.06.2014
(73) Proprietor: ABX Advanced Biochemical Compounds GmbH, 01454 Radeberg (DE)
(72) Inventor: Hoepping, Alexander, 01324 Dresden (DE); Müller, Marco, 01309 Dresden (DE); Smits, René, 01097 Dresden (DE); Mollitor, Jan, 01127 Dresden (DE); Clausnitzer, Andreas, 01454 Radeberg (DE); Baumgart, Diana, 01454 Ullersdorf (DE)
(74) Representative: Vossius & Partner Patentanwälte Rechtsanwälte mbB

(56) References cited:
- WO-A1-2010/146488
- WO-A2-2010/008522
- US-A1- 2004 034 246
- F. M. WAGNER ET AL: "Three-Step, "One-Pot" Radiosynthesis of 6-Fluoro-3,4-Dihydroxy-L-Phenylalanine by Isotopic Exchange", THE JOURNAL OF NUCLEAR MEDICINE, vol. 50, no. 10, 1 October 2009 (2009-10-01), pages 1724-1729, XP55060247, ISSN: 0161-5505, DOI: 10.2967/jnumed.109.063297

## Description

The invention relates to precursors for the production of optically active ¹⁸F-labelled amino acids bearing the radiochemical label on an aromatic ring. Examples for such amino acids are 6-[¹⁸F]Fluoro-3,4-dihydroxy-L-phenylalanine ([¹⁸F]F-L-DOPA) as well as para-[¹⁸F]Fluoro-L-tyrosine. Further, it relates to a radiochemical method for the production of the corresponding radiotracers.

Particularly [¹⁸F]F-L-DOPA has been used routinely for many years in positron-emission-tomography. Main fields of application are diagnosis of Parkinson's disease, hyperinsulinism in children and diagnosis of neuroendocrine tumors.

However, the application of [¹⁸F]F-L-DOPA is limited to a few specialized PET-centers, able to produce [¹⁸F]F₂. The [¹⁸F]F₂ is reacted in an electrophilic substitution reaction with a stannylated precursor to yield [¹⁸F]F-L-DOPA. This process provides the radiotracer only with moderate total activity. Furthermore, the specific activity is low due to the fact that only 50 % of the fluorine atoms in [¹⁸F]F₂ can be radioactive. The remaining 50 % are "cold", i.e. nonradioactive, fluorine. In addition to this, cold fluorine is added (carrier added, c.a.) to the [¹⁸F]F₂ in order to transport the radioactive gas and to make it available for the synthesis. Consequently, the specific activity will be further decreased.

The widespread use of [¹⁸F]F-L-DOPA depends on its efficient production. The radiotracer thus should be produced in high radiochemical yield and purity with a high total activity and a high specific activity. In view of the radiochemical half-life of ¹⁸F of 109.7 minutes, it will be understood in this respect that a high activity requires the synthesis of the radiotracer to proceed quickly. Otherwise, a large percentage of the activity is lost during the preparation of the labelled amino acids. If these requirements are met, several patients could be scanned with one batch of radiotracer. Additionally, a high specific activity keeps the applied dosis for each patient as low as possible.

Accordingly, there is a high interest to improve and automate the process to synthesize [¹⁸F]F-L-DOPA. One approach which is currently pursued is the further improvement of the electrophilic pathway. Many of the above mentioned problems, however, cannot be solved using this approach.

In contrast to this, the synthesis via a nucleophilic substitution reaction offers the advantage of using [¹⁸F]Fluoride. [¹⁸F]Fluoride can be produced in all cyclotrons for medical use worldwide in high total activity. However, most nucleophilic syntheses for [¹⁸F]F-L-DOPA require a multistep procedure. Radiolabeling is usually performed at an early stage of the total process. The labelled intermediate is then carried on and further steps are required to convert it into the final product. Automation of multistep procedures is not easy because of the complicated module design involved. Moreover, total activity will be lower in comparison to relatively short syntheses, or a very high starting activity needs to be used (R. N. Krasikova, V. V. Zaitsev, S. M. Ametamey, O. F. Kuznetsova, O. S. Fedorova, I. K. Mosevich, Y. N. Belokon, S. Vyskocil, S. V. Shatik, M. Nader, P. A. Schubinger, Nucl. Med. Biol. 2004, 31, 597; C. Lemaire, M. Guillaume, R. Cantineau, L. Christiaens, J. Nucl. Med. 1990, 31, 1247; C. Lemaire, P. Damhaut, A. Plenevaux, D. Comar, J. Nucl. Med. 1994, 35, 1996, C. Lemaire, S. Gillet, S. Guillouet, A. Plenevaux, J. Aerts, A. Luxen, Eur. J. Org. Chem. 2004, 2899; L. Zhang, G. Tang, D. Yin, X. Tang, Y. Wang, Appl. Radiot. Isot. 2002, 57, 145). WO 2010/008522 A2 describes iodylbenzene derivatives substituted with electron donating as well as electron withdrawing groups on the aromatic ring.

US2004/0034246 discloses methods for the synthesis of radiolabeled fluorinated compounds. Electron withdrawing groups in ortho- or para-position to the leaving group facilitate the nucleophilic aromatic substitution with [¹⁸F]Fluoride. Activating groups can be cleaved after the synthesis or transformed into substituents that are part of the molecule. Both possibilities are described in literature. A very promising approach is the activation using carbonyl groups. After the fluorination reaction, the carbonyl groups are transformed into hydroxy groups by Bayer-Villiger-oxidation. One example was published by Mulholland in US 2004/0034246. A further example was published by Coenen et al. (F. M. Wagner, J. Ermert, H. H. Coenen, J. Nucl. Med. 2009, 50, 1724). The authors describe a precursor for radiofluorination that is activated in para-position by a carbonyl group. The enantiospecific synthesis of the precursor was performed by an alkylation of the Seebach-auxiliary (*S*)-BocBMI ((*S*)-(-)-1-Boc*-*2*-tert-*butyl-3-methyl-4-imidazolidinone). The precursor contains a fluorine as a leaving group. This fluorine can be substituted with [¹⁸F]fluoride in good yields. The following Bayer-Villiger-oxidation proceeds with sufficient yields. However, the final deprotection of the labelled intermediate requires concentrated hydrobromic acid and high temperatures. These drastic conditions can lead to a partial racemisation of the final product and prevent the application on automated synthesis modules. Furthermore, plastic parts like tubings and valves are not compatible with these harsh reaction conditions and will decompose. This fact leads to low yields and poses a high risk for unintended release of radioactivity. Another disadvantage of this synthesis is the isotopic exchange of cold fluorine by hot fluorine that leads to only moderate specific activities.
It is an object of the invention to overcome the above disadvantages of the prior art. In particular a process for the preparation of precursors for [¹⁸F]F-L-DOPA and para-[¹⁸F]Fluor-L-tyrosine as well as a process for the radiosynthesis of said compounds is provided. The invention will allow for the automated synthesis of n.c.a. [¹⁸F]F-L-DOPA and para-[¹⁸F]Fluor-L-tyrosine with high radiochemical yield and high enantiomeric purity by employing mild acids at moderate temperatures for deprotection and suitable solvents for each reaction step.

Thus, according to a first aspect of the invention, there is provided a compound of formula (I) 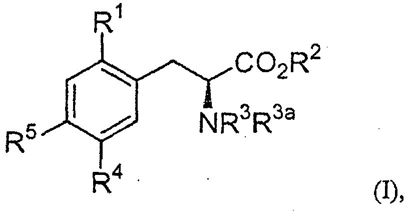 wherein
R¹ represents a nitro group;
R² represents tert-butyl;
R³ and R^{3a} independently represent hydrogen, or a group selected from -CO₂R⁶, -COR⁷ and - R⁸, wherein R⁶ is selected from C₁-C₆ alkyl which may be substituted, C₂-C₆ alkenyl which may be substituted and aralkyl which may be substituted, R⁷ is selected from hydrogen or C₁-C₆ alkyl which may be substituted, and R⁸ is selected from C₁-C₆ alkyl which may be substituted, C₂-C₆ alkenyl which may be substituted, and aralkyl which may be substituted;
R⁴ represents -COR⁹, wherein R⁹ is H;
R⁵ represents -OR¹⁰, wherein R¹⁰ is a linear or branched ether group of the formula -(C₁-C₆ alkyl)-O-(C₁-C₆alkyl) which can be substituted with a group -O(C₁-C₄ alkyl);
and wherein one or more of hydrogen atoms present in any alkyl or alkylene moiety in the compound of formula I can be substituted by deuterium.

In further aspects, the invention relates to the use of a compound of formula (I) (including its preferred embodiments) as a precursor for the production of a ¹⁸F-labelled amino acid in particular selected from 6-[¹⁸F]Fluoro-3,4-dihydroxy-L-phenylalanine ([¹⁸F]F-L-DOPA) and para-[¹⁸F]Fluoro-L-tyrosine.

Furthermore, the invention provides methods for the synthesis of a ¹⁸F-labelled amino acid, in particular selected from 6-[¹⁸F]Fluoro-3,4-dihydroxy-L-phenylalanine ([¹⁸F]F-L-DOPA) and para-[¹⁸F]Fluoro-L-tyrosine, said methods involving a nucleophilic substitution of the leaving group R¹ in a compound of formula (I) (including its preferred embodiments) by [¹⁸F]fluoride. Advantageously, these processes can be carried out as no-carrier-added (n.c.a.) syntheses. Thus, the resulting ¹⁸F-labelled amino acid contains the radiolabel attached to an aromatic ring in the position where group R¹ is present in the compound of formula (I) acting as a precursor or starting material in the concerned methods. In accordance with the method of the invention,the ¹⁸F-labelled amino acid can be obtained as an optically active compound, and in particular a high sterechemical purity of the target compound can be ensured.

As used herein, the term "C₁-C₆ alkyl" relates to a straight-chain or branched, saturated, aliphatic hydrocarbon group with 1 to 6 carbon atoms. Examples of C₁-C₆ alkyl groups are methyl, ethyl, n-propyl, iso-propyl, n-butyl, iso-butyl, sec-butyl, and tert-butyl, n-pentyl, n-hexyl.

Unless defined otherwise in any specific context, suitable substituents for C₁-C₆ alkyl are one or more, such as one, two or three substituents, selected from the group consisting of halogen, C₁-C₄ alkyl, -OH, -O(C₁-C₄ alkyl), -O-(C₁-C₄ alkyl)-O-(C₁-C₄ alkyl), -NO₂, -CN, -CO₂H and -CO₂(C₁-C₄ alkyl), wherein each of the preceding C₁-C₄ alkyl groups is unsubstituted or substituted with at least one halogen atom. For the optional halogen substitution, both in the case of a C₁-C₆ alkyl or any C₁-C₄ alkyl, it is preferred that the halogen is chloro or fluoro, and that the halogen substituted alkyl group is perchlorinated or perfluorinated, such as a trichloromethyl or trifluoromethyl group. Preferred substituted C₁-C₆ alkyl groups are trichloromethyl or trifluoromethyl, or carry a -O(C₁-C₄ alkyl) substituent, wherein the C₁-C₄ alkyl group is unsubstituted or substituted with at least one halogen atom, preferably unsubstituted. A particularly preferred optional substitutent is -O(C₁-C₄ alkyl).

As used herein, the term "C₂-C₆ alkenyl" relates to a straight-chain or branched, non-saturated, aliphatic hydrocarbon group containing one or more, preferably one, C-C double bond and 2 to 6 carbon atoms. An example of a C₂-C₆ alkenyl group is an allyl group.

Unless defined otherwise in any specific context, suitable substituents for C₂-C₆ alkenyl are one or more, such as one, two or three substituents, selected from the group consisting of halogen, C₁-C₄ alkyl, -OH, -O(C₁-C₄ alkyl), -NO₂, -CN, -CO₂H and -CO₂(C₁-C₄ alkyl), wherein each of the preceding C₁-C₄ alkyl groups is unsubstituted or substituted with at least one halogen atom. A preferred is -O(C₁-C₄ alkyl), wherein the C₁-C₄ alkyl group is unsubstituted or substituted with at least one halogen atom, preferably unsubstituted. For the optional halogen substitution of a C₁-C₄ alkyl group, it is preferred that the halogen is chloro or fluoro, and that the halogen substituted alkyl group is perchlorinated or perfluorinated, such as a trichloromethyl or trifluoromethyl group.

As used herein, the term "aralkyl" relates to an alkyl group, preferably a C₁-C₆ alkyl group as defined herein and most preferably a methyl group, wherein one or more, preferably one, two or three, hydrogen atoms are replaced by an aryl group. Preferred examples of the aryl group are selected from phenyl, naphtyl and 9-fluorenyl. Unless indicated otherwise in a given context, preferred examples of an aralkyl group are selected from a benzyl group, a 9-fluorenylmethyl group and a triphenylmethyl group. Unless defined otherwise in any specific context, a substituted aralkyl group can carry one or more substituents on its alkyl moiety, and/or one or more substituents on its aryl group(s).

Suitable substituents for the alkyl part of the aralkyl moiety are one or more, such as one, two or three substituents, selected from the group consisting of halogen, -OH, -O(C₁-C₄ alkyl), - NO₂, -CN, -CO₂H and -CO₂(C₁-C₄ alkyl), wherein each of the preceding C₁-C₄ alkyl groups is unsubstituted or substituted with at least one halogen atom, preferably unsubstituted. A preferred substituent for the alkyl moiety is -O(C₁-C₄ alkyl), wherein the C₁-C₄ alkyl group is unsubstituted or substituted with at least one halogen atom, preferably unsubstituted. For the optional halogen substitution of a C₁-C₄ alkyl group, it is preferred that the halogen is chloro or fluoro, and that the halogen substituted alkyl group is perchlorinated or perfluorinated, such as a trichloromethyl or trifluoromethyl group. More preferably, the alkyl moiety of the aralkyl group does not carry further substituents in addition to the aryl group(s).

Suitable substituents for any aryl part of the aralkyl moiety are one or more, such as one, two or three substituents, selected from C₁-C₄ alkyl, -OH, -O(C₁-C₄ alkyl), -CO₂H and -CO₂(C₁-C₄ alkyl), wherein each of the preceding C₁-C₄ alkyl groups is unsubstituted or substituted with at least one halogen atom, preferably unsubstituted. Preferred substituents for the aryl moiety are selected from the group consisting of C₁-C₄ alkyl and -O(C₁-C₄ alkyl), wherein the C₁-C₄ alkyl group is unsubstituted or substituted with at least one halogen atom, preferably unsubstituted. For the optional halogen substitution of a C₁-C₄ alkyl group, it is preferred that the halogen is chloro or fluoro, and that the halogen substituted alkyl group is perchlorinated or perfluorinated, such as a trichloromethyl or trifluoromethyl group. Preferred examples of substituted aralkyl groups are methoxybenzyl, such as p-methoxybenzyl, dimethoxybenzyl, methoxytrityl und dimethoxytrityl.

The term "C₁-C₆ alkanoyl" refers to a group of the formula -C(O)R¹², wherein R¹² is a C₁-C₆ alkyl group. In instances where the alkanoyl group is substituted, one or more, such as one, two or three substituents can be attached to R¹². The substituents are selected from the group consisting of halogen, C₁-C₄ alkyl, -OH, -O(C₁-C₄ alkyl), -O-(C₁-C₄ alkyl)-O-(C₁-C₄ alkyl), - NO₂, -CN, -CO₂H and -CO₂(C₁-C₄ alkyl), wherein each of the preceding C₁-C₄ alkyl groups is unsubstituted or substituted with at least one halogen atom, preferably unsubstituted. For the optional halogen substitution of the C₁-C₆ alkyl group of R¹², it is preferred that the halogen is chloro or fluoro, and that the halogen substituted alkyl group is perchlorinated or perfluorinated, such as a trichloromethyl or trifluoromethyl group. Preferred substituted groups R¹² are trichloromethyl or trifluoromethyl, or carry a -O(C₁-C₄ alkyl) substituent, wherein the C₁-C₄ alkyl group is unsubstituted or substituted with at least one halogen atom. For the optional halogen substitution of a C₁-C₄ alkyl group, it is preferred that the halogen is chloro or fluoro, and that the halogen substituted alkyl group is perchlorinated or perfluorinated, such as a trichloromethyl or trifluoromethyl group. Preferred are unsubstituted alkanoyl groups, unless indicated otherwise in a specific context.

The term "C₁-C₆ alkyloxycarbonyl" refers to a group of the formula -C(O)OR¹³, wherein R¹³ is a C₁-C₆ alkyl group. In instances where the alkyloxycarbonyl group is substituted, one or more, such as one, two or three substituents can be attached to R¹³. The substituents are selected from the group consisting of halogen, C₁-C₄ alkyl, -OH, -O(C₁-C₄ alkyl), -O-(C₁-C₄ alkyl)-O-(C₁-C₄ alkyl), -NO₂ , -CN, -CO₂H and -CO₂(C₁-C₄ alkyl), wherein each of the preceding C₁-C₄ alkyl groups is unsubstituted or substituted with at least one halogen atom, preferably unsubstituted. For the optional halogen substitution of the C₁-C₆ alkyl group of R¹³, it is preferred that the halogen is chloro or fluoro, and that the halogen substituted alkyl group is perchlorinated or perfluorinated, such as a trichloromethyl or trifluoromethyl group. Preferred substituted groups R¹³ are trichloromethyl or trifluoromethyl, or carry a -O(C₁-C₄ alkyl) substituent,, wherein the C₁-C₄ alkyl group is unsubstituted or substituted with at least one halogen atom. For the optional halogen substitution of a C₁-C₄ alkyl group, it is preferred that the halogen is chloro or fluoro, and that the halogen substituted alkyl group is perchlorinated or perfluorinated, such as a trichloromethyl or trifluoromethyl group. Preferred are unsubstituted alkyloxycarbonyl groups, unless indicated otherwise in a specific context.

The term "linear or branched ether" refers to linear or branched group containing an ether bond, preferably a group containing a of the formula -(C₁-C₆ alkyl)-O-(C)-C₆ alkyl), wherein the C₁-C₆ alkyl groups, which can be linear or branched, are as defined above. In instances where the linear or branched ether group is substituted, either one or both of the alkyl groups may carry one or more, such as one, two or three substituents. The substituents are selected from the group consisting of halogen, C₁-C₄ alkyl, -OH, -O(C₁-C₄ alkyl), -NO₂, -CN, -CO₂H and -CO₂(C₁-C₄ alkyl), wherein each of the preceding C₁-C₄ alkyl groups is unsubstituted or substituted with at least one halogen atom, preferably unsubstituted. For the optional halogen substitution of the C₁-C₆ alkyl group of R¹³, it is preferred that the halogen is chloro or fluoro, and that the halogen substituted alkyl group is perchlorinated or perfluorinated, such as, depending on the position a trichloromethyl/dichloromethylene or trifluoromethyl difluoromethylene group. Preferred substituents are selected from the group consisting of halogen, -OH, and -O(C₁-C₄ alkyl), wherein the C₁-C₄ alkyl group is unsubstituted or substituted with at least one halogen atom. For the optional halogen substitution of a C₁-C₄ alkyl group, it is preferred that the halogen is chloro or fluoro, and that the halogen substituted alkyl group is perchlorinated or perfluorinated, such as a trichloromethyl or trifluoromethyl group. Preferred are unsubstituted linear or branched ether groups, unless indicated otherwise in a specific context

The term "cyclic ether" refers to a cyclic group containing an ether bond, preferably a heterocyclic 5- or 6 membered cyclic group containing one or two oxygen atoms, or an oxygen atom and a nitrogen atom or an oxygen atom and a sulfur atom, wherein any two heteroatoms are not adjacent to each other. More preferred as a cyclic ether group is a group containing one oxygen atom as the only heteroatom, and particularly preferred is a tetrahydropyranyl ring, e.g. a tetrahydropyran-2-yl ring. In instances where the cyclic ether group is substituted, one or more, such as one, two or three substituents may be attachted to the ring and may replace each a hydrogen atom attached to a ring member. The substituents are selected from the group consisting of halogen, C₁-C₄ alkyl, -OH, -O(C₁-C₄ alkyl), -NO₂, - CN, -CO₂H and -CO₂(C₁-C₄ alkyl), wherein each of the preceding C₁-C₄ alkyl groups is unsubstituted or substituted with at least one halogen atom, preferably unsubstituted. For the optional halogen substitution of the cyclic ether, it is preferred that the halogen is chloro or fluoro, and that the halogen substituted group is perchlorinated or perfluorinated. Preferred as a substituent is -O(C₁-C₄ alkyl), wherein the C₁-C₄ alkyl group is unsubstituted or substituted with at least one halogen atom. For the optional halogen substitution of a C₁-C₄ alkyl group, it is preferred that the halogen is chloro or fluoro, and that the halogen substituted alkyl group is perchlorinated or perfluorinated, such as a trichloromethyl or trifluoromethyl group. Preferred are unsubstituted cyclic ether groups, unless indicated otherwise in a specific context.

The term "nitro" relates to the group NO₂ bound to a carbon atom.

The term "halogen" relates to F, Cl, Br, or I, preferably F, Cl or Br.

R¹ in the compounds of the invention is a nitro-group. It has surprisingly been found that precursors of formula (I) wherein R¹ is a nitro group yield ¹⁸F-labelled amino acids with a particularly high activity after the nucleophilic substitution of R¹ with [¹⁸F] fluoride.

R² in the compounds of the invention is *tert*-butyl.

It is generally preferred for the compounds of the invention that R³ and R^{3a} are not both hydrogen at the same time, in which case one of R³ and R^{3a} can act as a protective group for the amino group in formula (I). It is more preferred that R^{3a} is H, and R³ is a group selected from -CO₂R⁶, -COR⁷ and -R⁸.

R⁶ is preferably selected from the group consisting of methyl, ethyl, propyl, iso-propyl tert-butyl, allyl, neopentyl, benzyl, and 9-fluorenylmethyl. More preferably, R⁶ is *tert-*Butyl*.*

R⁷ is preferably selected from the group consisting of hydrogen, methyl and trifluoromethyl. More preferably, R⁷ is hydrogen.

R⁸ is preferably selected from the group consisting of benzyl, methoxybenzyl, dimethoxybenzyl, allyl, diphenyl, triphenylmethyl (trityl), methoxytrityl and dimethoxytrityl. More preferably, R⁸ is trityl.

Taking into account the above definitions of R³, R^{3a} and the groups R⁶, R⁷ and R⁸ which may be contained therein, it is more preferred that R^{3a} is hydrogen and R³ is -CO₂R⁶, or -R⁸, in particular -R⁸. R⁸ can have the meanings indicated above, but it is preferred in this context that -R⁸ is aralkyl which may be substituted. More preferably, R⁸ is selected from trityl, methoxytrityl and dimethoxytrityl, and in particular it is trityl.

R¹⁰, which can be considered as a protective group for a hydroxy group, is a linear or branched ether group of the formula -(C₁-C₆ alkyl)-O-(C₁-C₆ alkyl) which may be substituted with a group -(C₁-C₄ alkyl). As specific preferred embodiments for R¹⁰, the following can be listed: methoxymethyl, 2-methoxyethoxymethyl, ethoxymethyl and ethoxyethyl. Particularly preferred as R¹⁰ is methoxymethyl.

R⁵ is -OR¹⁰.

Thus, in accordance with one particularly preferred embodiment of the invention, the compounds of formula (I) are those of formula (Ia): 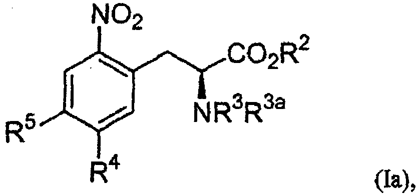 wherein the definitions of R², R³, R^{3a}, R⁴ and R⁵, including preferred definitions provided herein, apply.

In accordance with a further preferred embodiment, the compounds of formula (I) are those of formula (Ib): 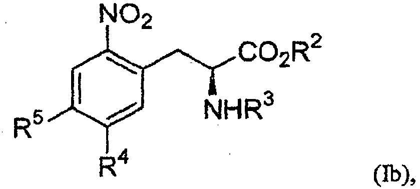 wherein the definitions of R², R³, R⁴ and R⁵, respectively, including preferred definitions provided herein, apply, with the proviso that R³ is not hydrogen.

In accordance with a further preferred embodiment, the compounds of formula (I) are those of formula (Ic): 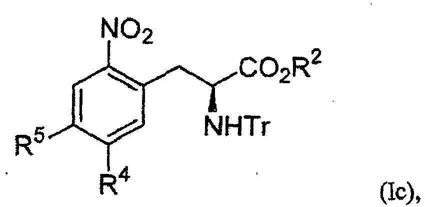 and in particular those of formula (Ic'): 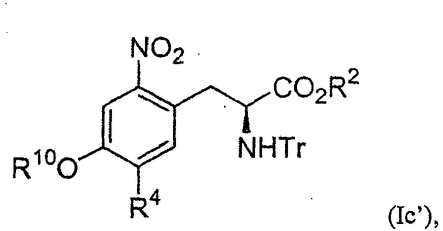 wherein the definitions of R², R⁴ and R⁵ or R¹⁰, respectively, including preferred definitions provided herein, apply, and Tr represents a triphenylmethyl (trityl) group.

In accordance with a particularly preferred embodiment, the compound of formula (I) is the 3-(5-formyl-4-methoxymethoxy-2-nitro-phenyl)-2-(trityl-amino)-propionic acid *tert*-butyl ester of formula (Id): 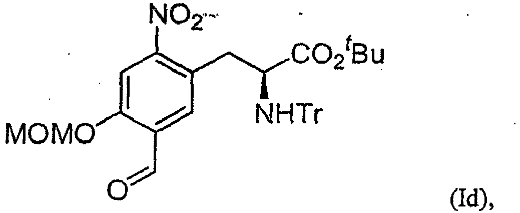 wherein Tr represents the trityl group and MOM represents the methoxymethyl group.

The compounds in accordance with the invention have at least one, frequently exactly one chiral center, the stereochemistry of which is indicated in formula (I) and the preferred embodiments thereof in accordance with established conventions. The compounds of formula (I) are preferably provided and used as precursors in a form which is stereochemically pure, i.e. consists of the stereoisomer shown in the formula in the absence of its stereochemical counterpart, or contains only trace amounts of the stereochemical counterpart. However, it will be understood that certain benefits of the invention may also be achieved if a compound of the invention is used in a mixture with the corresponding isomer wherein the stereochemical configuration indicated in the formulae of the invention is inversed. In such a case, the enantiomeric excess (e.e.) of the compound in accordance with the invention in such a mixture is preferably 80 % or more, more preferably 90 % or more, in particular 95 % or more, and most preferably 99 % or more.

As noted above, the compounds of the invention as defined herein can be conveniently used as a precursor for the preparation of a ¹⁸F-labelled amino acid which contains the radiolabel (i.e. the ¹⁸F-atom) attached to an aromatic ring. The labelled amino acid can be provided with a high specific activity, especially since the precursor can be converted in a no-carrier-added synthesis. Moreover, the total reaction time is reduced and the deprotection of the labelled precusor can be carried out under mild reaction conditions. The labelled amino acid can be obtained in high yields and in high chemical, enantiomeric and radiochemical purity.

Preferred ¹⁸F-labelled amino acids which can be prepared using the precursor compounds of the present invention are selected from:
[¹⁸]F-L-DOPA of the following formula 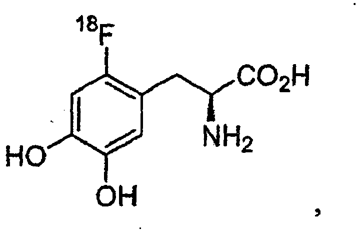
and *para*-[¹⁸P]fluoro-L-tyrosine of the formula

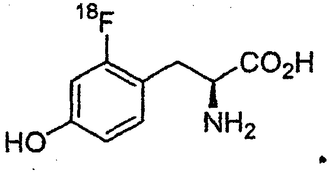

For the preparation of ¹⁸F]F-L-DOPA and of *para*-[¹⁸F]fluoro-L-tyrosine, a precursor of formula (I) (or its preferred embodiments) wherein R⁵ is -OR¹⁰ is used.

As used herein, the reference to an ¹⁸F-labelled amino acid or to one of the preferred embodiments thereof includes salt forms, in particular pharmaceutically acceptable salts as they are known in the art, such as the internal salt form of the amino acid, a base addition salt formed via deprotonation of the carboxyl group, or an acid addition salt formed via protonation of the amino group with an acid. Exemplary base addition salts comprise, for example, alkali metal salts such as sodium or potassium salts; alkaline-earth metal salts such as calcium or magnesium salts; ammonium salts; aliphatic amine salts such as trimethylamine, triethylamine, dicyclohexylamine, ethanolamine, diethanolamine, triethanolamine, procaine salts, meglumine salts, diethanol amine salts or ethylenediamine salts; aralkyl amine salts such as N,N-dibenzylethylenediamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts or lysine salts. Exemplary acid addition salts comprise, for example, mineral acid salts such as hydrochloride, hydrobromide, hydroiodide, sulfate salts, nitrate salts, phosphate salts (such as, e.g., phosphate, hydrogenphosphate, or dihydrogenphosphate salts), carbonate salts, hydrogencarbonate salts or perchlorate salts; organic acid salts such as acetate, propionate, butyrate, pentanoate, hexanoate, heptanoate, octanoate, cyclopentanepropionate, undecanoate, lactate, maleate, oxalate, fumarate, tartrate, malate, citrate, nicotinate, benzoate, salicylate or ascorbate salts; sulfonate salts such as methanesulfonate, ethanesulfonate, 2-hydroxyethanesulfonate, benzenesulfonate, p-toluenesulfonate (tosylate), 2-naphthalenesulfonate, 3-phenylsulfonate, or camphorsulfonate salts; and acidic amino acid salts such as aspartate or glutamate salts.

In accordance with the above, the present invention further provides a method for the synthesis of a ¹⁸F-labelled amino acid which contains the radiolabel attached to an aromatic ring, said method comprising the steps of
a) providing or obtaining a compound of formula (I) (including its preferred embodiments as defined herein, in particular a compound of formula (Ia), (Ib), (Ic), (Ic') or more preferably (Id)), and
b) carrying out a nucleophilic substitution of the leaving group R¹ in the compound of formula (I) by [¹⁸F]fluoride.

Advantageously, the method is a no-carrier-added synthesis, i.e. no unlabelled (i.e. nonradioactive or "cold") fluorine containing compound is added as a carrier for the labelled amino acid.

When using the method in accordance with the present invention, the ¹⁸F-labelled amino acids as target compounds are obtained as optically active compounds, and it is an advantage in this respect that a high stereochemical purity can be ensured. Preferably, the stereochemistry of the compound of formula (I) or its preferred embodiments as a starting compound, as indicated by the stereochemistry in the formula, is retained to a maximum degree and racemisation is avoided. Thus, for example, if a starting compound of formula (I) is used which is stereochemically pure or contains only trace amounts of an additional isomer, the labelled amino acid is afforded in high enantiomeric purity of 95 % or more, preferably 98 % or more, and particularly > 99%, wherein the percentage relates to the ratio of the desired enantiomer in the total amount of the enantiomers.
The [¹⁸F]fluoride to be used for the nucleophilic substitution can be prepared according to methods known in the art, e.g. by the ¹⁸O(p,n)¹⁸F nuclear reaction in a cyclotron target body (e.g. Coenen et al.,J. S. Fowler, A. P. Wolf. Nucl. Sci. Ser. Natl Acad. Sci. Natl Res. Council Monogr. 1982). After trapping an aqueous solution of [¹⁸F]fluoride on an anion exchange cartridge, the obtained [¹⁸F]fluoride can be eluted with bases like potassium carbonate or cesium carbonate and/or phase transfer catalysts (TBA and TEA) into a reaction vial. Then, the solution can be evaporated by azeotropic distillation, e.g. with acetonitrile.

The dried [¹⁸F]fluoride/phase transfer catalyst system can then be used for radiofluorination of a compound of formula (I) e.g. in an organic solvent. The nucleophilic substitution reaction to replace the leaving group R¹ by [¹⁸F]fluoride can be accomplished under conditions known in the art. Reference is made, e.g., to the methods for nucleophilic radiofluorination discussed in US 7,115,766 and the literature cited therein. For example, procedures using phase transfer catalysts like cryptands, tetra-n-butyl ammonium (TBA), tetra-n-ethyl ammonium (TEA) and others can be used. Preferred in the context of the present invention is a reaction using cryptand 222 or TBA as a phase transfer catalyst. Most preferred is TBA. Preferred solvents are acetonitrile, DMF, DMSO, toluene and ionic liquids. Most preferred is DMSO. The reaction can be conveniently carried out, e.g. in DMSO, at temperatures between 90 and 110°C.

Exemplary amounts of the precursor of formula (I) or its preferred embodiments which are subjected to the nucleophilic radiofluorination reaction depend on the reaction equipment. In regular laboratory devices, the amounts frequently range between 1 to 100 mg, preferably 1 to 30 mg. By using microfluidic devices, amounts of 1 ng to 1 mg can be subjected to the reaction. The ¹⁸F-substituted product can be trapped after the reaction on a solid phase cartridge, and can be subsequently purified by known methods as needed.

Furthermore, in one preferred embodiment, the method for the synthesis of a ¹⁸F-labelled amino acid of the invention comprises, subsequent to step b) a step c1) of converting R⁴ to an formyl ester (-OC(O)H) group. For example, the step c1) can be accomplished by oxidizing the group R⁴ under Bayer-Villiger conditions to form the formyl ester, which is preferably hydrolyzed at a later stage of the preparation of the ¹⁸F-labelled amino acid to yield a group - OH at the respective position which is occupied by R⁴ in formula (I).

The Bayer-Villiger oxidation can be performed with an oxidizing agent such as hydrogen peroxide, meta-chloroperoxyperbenzoic acid (mCPBA), Magnesium bis(monoperoxyphthalate) (MMPP), peracetic acid, periodic acid or perborate. Most preferred is mCPBA. The amount of the oxidizing agent, such as the mCPBA, generally ranges from 1 to 100mg in 100-5000 µL of solvent. The oxidation can be conveniently performed at temperatures between 50 and 100 °C. Suitable solvents include halogenated hydrocarbons like chloroform or dichloromethane, acetonitrile, methanol or ethanol. Most preferred is acetonitrile or ethanol. However, the use of halogenated hydrocarbons may not be compatible with the plastic parts of many synthesis modules used in automatic synthesis. Another drawback is the toxicity of such solvents, such that the use of such solvents should be limited for the production of pharmaceutical products (European Pharmacopoeia).

In another preferred embodiment, the method for the synthesis of a ¹⁸F-labelled amino acid of the invention comprises, subsequent to step b) a step c2) of replacing the group R⁴ by a hydrogen atom via decarbonylation. The decarbonylation of ¹⁸F-labelled compounds can be mediated by metal catalysts (e.g. B. Shen, D. Löffler, K.-P. Zeller, M. Übele, G. Reischl, H.-J. Machulla, App. Rad. Iso. 2007, 65, 1227).Suitable catalysts for the decarbonylation comprise one or more transition metals of the secondary groups 11 (or Ib), 12 (or IIb) 4 (IVb), 6(VIb), 8, 9 or 10 (VIIIb) of the periodic system such as chromium, manganese, nickel, copper or zinc, and preferably one or more metals from the group consisting of the platinum metals, in particular rhodium. Solid catalysts on carriers may be used as heterogeneous catalysts. Alternatively, the catalysts can be used in a homogeneous system in the liquid phase. Suitable rhodium complexes, which are soluble and can be used as homogeneous catalysts or which can be impregnated onto a carrier, are, for example, rhodium(I) complexes such as ClRh(PPh₃)₃ ("Wilkinson catalyst"), ClRh(CO) (PPh₃)₂, [ClRh(CO)₂]₂, acacRh(CO)(PPh₃), acacRh(CO)₂, (C₅H₅)Rh(C₈H₁₄) and (C₃H₅)Rh(PPh₃), where Ph is phenyl, acac is acetylacetonate, C₈H₁₄ is cyclooctene, C₅H₅ is cyclopentadienyl, and C₃H₅ is allyl. Rhodium(II) and rhodium(III) complexes can also be used, such as rhodium(II)-acetate, rhodium(II)-2,4-difluorobenzoate, Rh(acac)₃, RhCl₃×H₂O, Rh(NO₃)₃ and (C₃H₅)RhCl₂(PPh₃)₂. Advantageously, compounds which can act as ligands, such as phosphanes, phosphites or amines, may be added to these rhodium complexes.

In order to obtain a compound containing a carboxylic group and/or an amino group (or a salt of one or both of these), it is further preferred that the method for the synthesis of a ¹⁸F-labelled amino acid of the invention comprises, subsequent to step b), the step(s) of
d1) removing the group R² or replacing it by hydrogen in order to obtain a free carboxyl group or a salt thereof, and/or
d2) replacing any group(s) R³ or R^{3a} other than hydrogen by hydrogen in order to obtain a free amino group or a salt thereof.

Similarly, in cases where R⁵ in the compound of formula (I) provided or obtained in step a) is a group -OR¹⁰, it is preferred that the method further comprises, subsequent to step b), a step d3) of replacing the group R¹⁰ by hydrogen in order to obtain a free hydroxyl group.

In accordance with preferred embodiments of the method in accordance with the invention, steps d1) and d2), and, if necessary, d3) are combined with step c1) or with step c2), and the steps d1), d2) and, if necessary, d3) are carried out subsequent to step c1) or step c2).

The removal or replacement of the groups R², R³, R^{3a} (if different from hydrogen) and/or R¹⁰ which act as protecting groups for the functional groups to which they are attached, can be accomplished via known methods for the removal of protecting groups in organic chemistry. In the present case, the groups R², R³, R^{3a} and/or R¹⁰ and in particular the preferred embodiments have been selected so as to allow the removal thereof via hydrolysis under acidic conditions. For example, hydrolysis is performed with concentrated or semiconcentrated acids or mixtures of acids and ethanol or acids and acetonitrile. Preferred acids are HCl, HI, or HBr.

Most preferred is the use of hydrochloric acid. Preferred is a concentration of 1 to 12M hydrochloric acidat temperatures between 30 and 120 °C, in particular between 40 and 80 °C. Most preferred is a concentration of 6M hydrochloric acid at 50°C. The use of higher concentrated acids and higher temperatures can lead to a significant degree of racemisation of the optically active precursor, e.g. to the formation of [¹⁸F]F-D-DOPA.

Therefore it is particularly preferred that the precursor of formula (I) (its preferred embodiments) bears protecting groups that can be cleaved under mild conditions. For example, a trityl protecting group R³ for protection at the amine function can be advantageously cleaved with semiconcentrated HCl at moderate temperatures as indicated above, e.g. 50 °C without racemisation, thus affording the labelled L-amino acid such as [¹⁸F]F-L-DOPA in high enantiomeric purity of > 99%. The use of a di-Boc protecting group at the amine yields 1-2% of the D-form, such as [¹⁸F]F-D-DOPA and the use of a Boc protecting group at the amine yields more than 3% of the D-form. Moreover, the possibilty to use dilute mineral acids facilitates the automation of the synthesis. In order to be able to simultaneously remove all protecting groups under mild conditions, it is preferred that also the protecting group for the carboxylic group can be removed under such conditions. Therefore, R² is *tert.*-butyl. R¹⁰ is preferably selected from methoxymethyl, ethoxymethyl, ethoxyethyl and 2-methoxyethoxymethyl. It is most preferred a methoxymethyl group.

In cases where the method for the synthesis of a ¹⁸F-labelled amino acid in accordance with the invention comprises the step c1), wherein a formyl ester is introduced to the position of R⁴ as described above, it is further preferable that the method further comprises:
a step d4), wherein the formyl ester is hydrolyzed to yield a hydroxyl group at the position where R⁴ is present in the compounds of formula (I) and its preferred embodiments. The hydrolysis of step d4) can be carried out, e.g., under the conditions described above for the removal of the protective groups in steps d1), d2) and/or d3).

It is possible to use groups R², R³, R^{3a} (if different from hydrogen) R¹⁰ and or the formyl ester (if present) with different degrees of sensitivity to hydrolyzation, such that the steps d1), d2), d3) and d4) of removing or replacing groups R², R³, R^{3a}, R¹⁰ and/or the formyl ester can carried out selectively and separate from each other. However, in terms of an efficient, fast synthesis, it is preferred that the steps d1) and d2), if necessary d3) and, if necessary d4) are carried out simultaneously in one hydrolysis reaction. Especially if the preferred groups R², R³, R^{3a} (if different from hydrogen), R¹⁰ defined above and/or the formyl ester (if present) are combined, this can be conveniently accomplished.

Further purification of the final labelled amino acid can be performed by known means, e.g. by solid phase purification or HPLC.

It is generally preferred in the method for the synthesis of a labelled amino acid in accordance with the invention that the method comprises:
- steps a), b), c1), d1), d2), d3) and d4) if R⁵ in the compound of formula (I) or its preferred embodiments as the starting compound in step a) is -OR¹⁰; or compound
- steps a), b), c2), d1), d2) and d3) if R⁵ in the compound of formula (I) or its preferred embodiments as the starting compound in step a) is -OR¹⁰.

If the above step c1) and one or more of the above steps d1), d2), d3) and d4) are combined in the method in accordance with the invention, it is preferred that the steps d1), d2), d3) and/or d4) are carried out subsequent to step c1).

If the above step c2) and one or more of the above steps d1), d2) and d3) are combined in the method in accordance with the invention, it is preferred that the steps d1), d2) and/or d3) are carried out subsequent to step c2).

Thus, in one further preferred embodiment of the method for the synthesis of a labelled amino acid in accordance with the invention, the synthesized ¹⁸F-labelled amino acid is [¹⁸F]Fluor-L-DOPA, and the method comprises
the step a), wherein the compound of formula (I), preferably (Ia), more preferably (Ib), even more preferably (Ic) or (Ic') and in particular (Id), has a group -OR¹⁰ at position R⁵,
the step b),
the step c1) subsequent to step b),
and the steps d1), d2), d3) and d4) carried out simultaneously subsequent to step c1).

In another particularly preferred embodiment of the method for the synthesis of a labelled amino acid in accordance with the invention, the synthesized ¹⁸F-labelled amino acid is *para-*[¹⁹F]Fluor-L-tyrosin, and the method comprises
the step a), wherein the compound of formula (I), preferably (Ia), more preferably (Ib), even more preferably (Ic) or (Ic'), and in particular (Id), has a group -OR¹⁰ at position R⁵,
the step b),
the step c2) subsequent to step b),
and the steps d1), d2) and d3) carried out simultaneously subsequent to step c2).

The application of the compounds of formula (I), particularly the preferred precursor of formula Ia, Ib, Ic, Ic' and Id affords for the first time [¹⁸F] radiolabelled amino acids such as [¹⁸F]F-L-DOPA under n.c.a conditions via a nucleophilic synthesis in only three radioactive steps with an enantiomerical purity of more then 98%. Radiochemical yield is about 20% (+/-5%). For the final cleavage of the protecting groups dilute mineral acids can be used for the first time. This lends the whole synthesis to automation.

In the following, steps of the method in accordance with the invention shall be discussed in further detail with a view to preferred, exemplary embodiments. However, this information is not intended to impose any limitation on the more general disclosure above.

### Ste b)

In accordance with this embodiment, the method according to the invention can comprise the reaction of a compound of formula Ib, wherein R⁵ is -OR¹⁰, with [¹⁸F]fluoride in order to obtain a compound of formula XII.

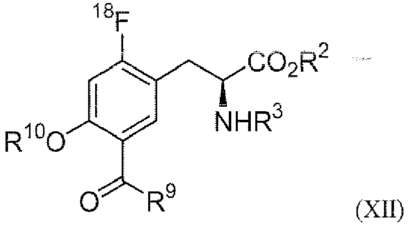

Compound of formula XII is obtained by nucleophilic substitution of a nitro group with ¹⁸F-fluoride in a compound of formula Ib. Surprisingly, the combination of a nitro group as leaving group and the trityl group as a preferred protecting group for the amine affords the product in a particularly good radiochemical yield and high specific activity.

### Step c1)

Further, the method according to the invention can comprise the reaction of a compound of formula XII in order to obtain a compound of formula XIII

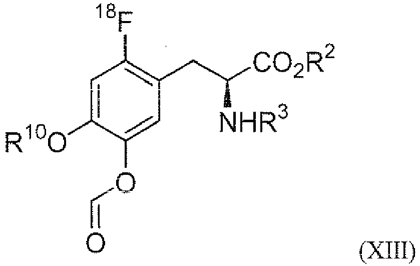

In accordance with this preferred step, the formyl group in the compound of formula XII is oxidized under Bayer-Villiger conditions to obtain the formyl ester of formula XIII. The Bayer-Villiger oxidation can be performed e.g. with hydrogen peroxide, mCPBA, MMPP, peracetic acid, periodic acid or perborate. Most preferred is mCPBA. The oxidation can be conveniently performed at temperatures between 50 and 100 °C. Most preferred is a temperature between 60 and 85°C.Preferred solvents are halogenated hydrocarbons like chloroform or dichloromethane or acetonitrile, methanol and ethanol.
Most preferred is acetonitrile or ethanol.

### Steps d1), d2), d3) and d4)

Further, the method according to the invention can comprise the reaction of a compound of formula XIII in order to obtain [¹⁸F]F-L-DOPA as a compound of formula XI.

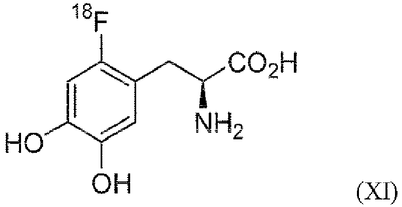

A compound of formula XIII can be hydrolyzed to [¹⁸F]F-L-DOPA, preferably directly after the removal of the oxidation agent used for the formation of the formyl ester, e.g. over a solid phase cartridge. Hydrolysis can be performed with concentrated or semiconcentrated acids or mixtures of acids and ethanol or acids and acetonitrile.

A particularly preferred embodiment of the method for the synthesis of a labelled amino acid in accordance with the invention, wherein the synthesized ¹⁸F-labelled amino acid is [¹⁸F]Fluor-L-DOPA, and is synthesized using a compound of formula (Id) is further illustrated in the following reaction scheme 1.

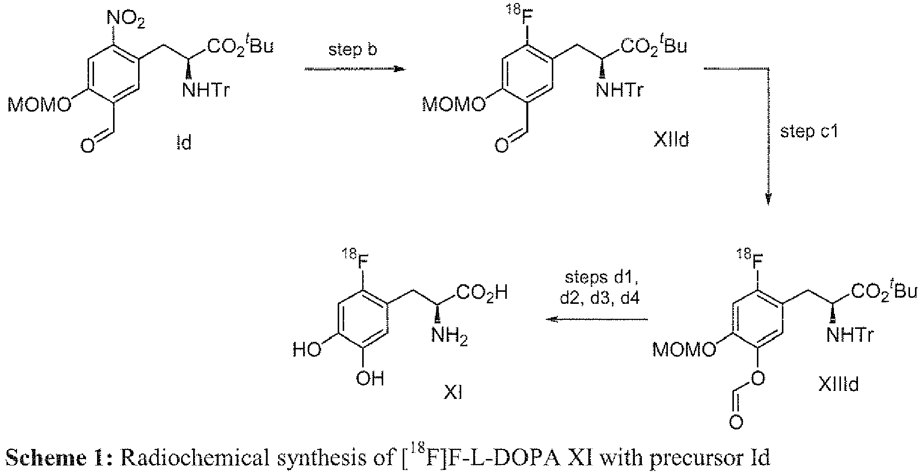

The compounds in accordance with the invention can be prepared by the skilled practitioner using known synthetic procedures. In the following, the synthesis of a preferred compound of formula I is explained in further detail.

### Step 1

The method according to the invention can comprise the reaction of a compound of formula II 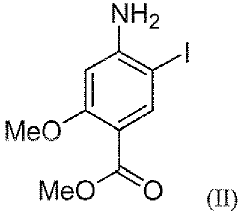 to provide a compound of formula III.

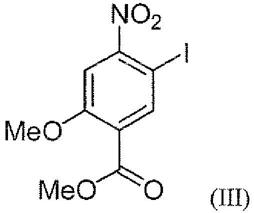

Compounds of formula II are accessible through syntheses described in the literature (e.g. J. Labelled Compd. Radiopharm. 2003, 46, 511-513).

A compound of formula II can be converted to a compound of formula III by oxidation of the aromatic amine. For the oxidation reagents like peracetic acid, manganese dioxide, lead tetraacetate, mercury(II)acetate, oxone, dioxirane and metal catalysts like molybdenum, tungsten, zirconium, rhenium, as well as titanium silicates, chromium silicates, iron(III)tetraarylporphyrins and manganese(III)tetraarylporphyrins in the presence of peroxides and catalytical amounts of iodide salts or cobalt-salen-complexes in the presence of peroxides can be used.

As an alternative route, the nitro group can be introduced by reacting a diazonium salt with sodium nitrite. Most preferred is the use of potassium iodide in the presence of of *tert.-*butylhydroperoxide. Employing this route affords a compound of formula III in high purity.

### Step 2

In a subsequent step, the methyl ether in a compound of formula III can be cleaved to yield a compound of formula IV.

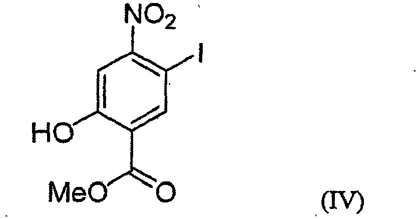

A compound of formula III is preferably converted in a compound of formula IV with a Lewis acid. Suitable Lewis acids are boron trichloride, boron tribromide, aluminium trichloride, manganese diiodide and iodotrimethylsilane. Most preferred is boron tribromide.

### Step 3

Further, the method for providing a compound of the invention may include the introduction of a protecting group into a compound of formula IV to obtain a compound of formula V, wherein R¹⁰ is defined as for formula (I), including preferred embodiments.

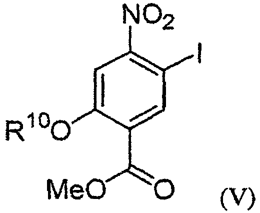

For the subsequent course of the synthesis and for the radiochemical production of [¹⁸F]F-L-DOPA the phenolic hydroxy group of compound of formula V should be protected with a suitable protecting group. Preferred are protecting groups which can be cleaved with mild acids. Exemplary protecting groups are methoxymethyl, 2-methoxyethoxymethyl, ethoxymethyl and ethoxyethyl. Most preferred is methoxymethyl.

### Step 4

Further, the method for providing a compound of the invention may include the reduction of a compound of formula V to obtain a compound of formula VI.

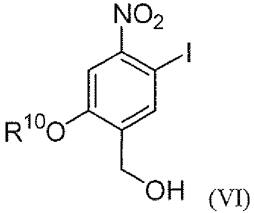

The ester group in a compound of formula V can be reduced to the corresponding benzyl alcohol to obtain a compound of formula VI. Preferably the reduction will be performed with metal hydrides. Most preferred are diisobutylaluminium hydride and lithium aluminium hydride.

### Step 5

Further, the method for providing a compound of the invention may include the Negishi-coupling of a compound of formula VI with an iodo alanine of formula VII to obtain a compound of formula VIII.

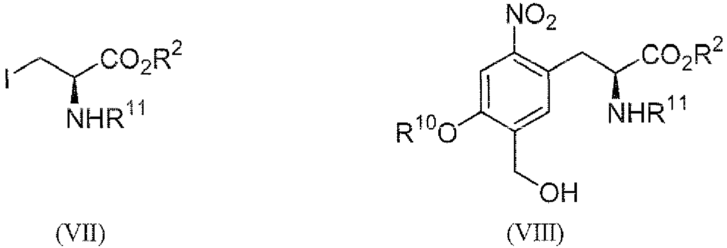

Palladium-catalyzed couplings of organozinc compounds of serine with aryl halogenides offer an efficient access to enantiomerically pure phenylalanines (Synlett 2005, 2701-2719). Thus, the synthesis of enantiomerically pure ortho-nitro-phenyl alanines can be accomplished via a Negishi-coupling of organozinc compounds of serine. However, until now, only moderate yields have been obtained (J. Org. Chem. 1998, 63, 7875-7884; Tetrahedron 2008, 64, 681-687). Recently, the method was improved and by using the ligand SPhos the yields could be increased (J. Org. Chem. 2010, 75, 245-248; J. Am. Chem. Soc. 2008, 126, 13028-13032). Most notably is that besides a great variety of functional groups also acidic protons are well tolerated. The use of this novel variety of the Negishi-coupling may now lead to compounds of formula VIII which can then be further transformed into precursors for the production of [¹⁸F]-labelled amino acids containing a nitro-group as leaving group. Furthermore, the method offers the possibility of introducing protecting groups suitable for the radiosynthesis.

A compound of formula VI can be reacted with iodo alanine of formula VII via the Negishi-coupling to a phenyl alanine of formula VIII. For the synthesis palladium catalysts are utilized.

Preferred are palladium(II)acetate and tris(dibenzylideneaceton)dipalladium. Suitable coligands are phosphines and arsines. Prefered are tri(*o*-tolyl)phosphine, SPhos and RuPhos.

Most prefered is SPhos. Prefered is a ratio of palladium to ligand of 2 to 1 and 1 to 1. Most preferred is a ratio of 1 to 1. Preferred is a catalyst load of 0,5 mol % to 5 mol % palladium. Most preferred is a catalyst load of 5 mol % palladium. Preferred is a ratio of iodo alanine to aryl iodide of 1 to 0,4 up to 1 to 1,3. Most preferred is a ratio of 1 to 0,4.

Syntheses of compounds of formula VII are known to the skilled person from the literature. (N-Fmoc-Iodalanin-*tert*.-butylester: J. Med. Chem. 2006, 49, 2200-2209; J. Chem. Soc, Perkin Trans 1 2001, 1876-1884; *N*-Boc-Iodalanin-*tert*.-butylester: J. Org. Chem. 1998, 63, 7875-7884; Angew. Chem. Int. Ed. 2011, 50, 7604-7606).

R² and R¹¹ are two additional protecting groups. A direct introduction of the most preferred trityl protecting group as R¹¹ is not preferred. N-trityl iodalanines tend to form aziridines, which can be opened to two regioisomeric products. (J. Org. Chem. 2003, 68, 8185-8192). This side reaction may also occur under Negishi-coupling conditions. Hence, if a trityl group is desired as the protecting group in the compound of formula (I), the protecting group R¹¹ should be cleaved after the Negishi-coupling in order to introduce the trityl group. R² is as defined above for formula (I), including preferred. R¹¹ is preferably selected from the group, consisting of Fmoc, allyl, allyloxycarbonyl, benzyl, methoxybenzyl, dimethoxybenzyl, nitrobenzyl, benzyloxycarbonyl. Most preferred is Fmoc as R¹¹ = Fmoc

### Step 6

Further, the the method for providing a compound of the invention may include the cleavage of the amine protecting group in a compound of formula VIII in order to obtain a compound of formula IX

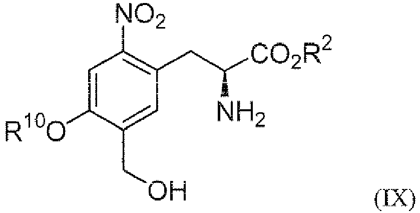

If R¹¹ is the most preferred group Fmoc, basic conditions can be used for cleavage. Preferred bases are ammonia, diethylamine, piperidine, morpholine and DBU. Most preferred are piperidine and morpholine.

### Step 7

Further, the method for providing a compound of the invention may include the protection of the amine function in a compound of formula IX in order to obtain a compound of formula X

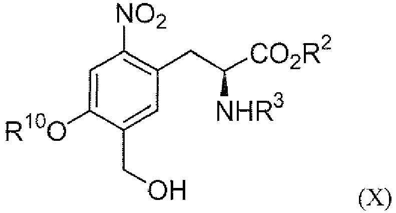

Thus, the amine function of a compound of formula X is protected with a protecting group R³ as defined for formula (I) above, including preferred embodiments. Preferred protecting groups can be cleaved under mild acidic conditions like *tert*.-butyloxycarbonyl, formyl, or trityl. Most preferred is R³ = trityl.

### Step 8

Further, the method for providing a compound of the invention may include the oxidation of a compound of formula X in order to obtain a compound of formula I as shown below, wherein R², R³ and R¹⁰ are as defined above, including preferred embodiments.

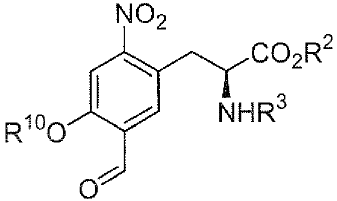

The alcohol function in a compound of formula X is oxidized to an aldehyde group to obtain the compound of formula I. Preferred oxidation agents are DMP, IBX, TPAP, oxalylchloride/DMSO, TEMPO, sulfur trioxide pyridine complex, MnO₂ or PCC. Most preferred is DMP.

Hereinafter, the invention is explained in more detail with the help of examples which are not intended to limit the scope of the invention.

### Examples

The abbreviations have the following meaning
F-L-DOPA = 6-Fluoro-3,4-dihydroxy-L-phenylalanine
c.a. = carrier-added
n.c.a. = no-carrier-added
BocBMI = (*S*)-(-)-1-Boc-2-*tert*-butyl-3-methyl-4-imidazolidinone
Trityl = Tr = triphenylmethyl
Boc = tert.-butyloxycarbonyl
MOM = methoxymethyl
TBA = tetra-n-butyl ammonium
TEA = tetra-n-ethyl ammonium
cryptand 222 = 4,7,13,16,21,24-Hexaoxa-1,10-diazabicyclo[8.8.8]-hexacosane
DMF = N,N-dimethylformamid
DMSO = dimethyl sulfoxide
mCPBA = meta-chloroperoxyperbenzoic acid
MMPP = magnesium bis(monoperoxyphthalate)
HPLC = high-performance liquid chromatography
SPhos = 2-Dicyclohexylphosphino-2',6'-dimethoxybiphenyl
RuPhos = 2-Dicyclohexylphosphino-2',6'-diisopropoxybiphenyl
SPE = solid phase extraction
Fmoc = fluorenylmethyloxycarbonyl
DBU = 1,8-diazabicyclo[5.4.0]undec-7-ene
DMP = Dess-Martin periodinane
IBX = 2- Iodoxybenzoic acid
TPAP = tetrapropylammonium perruthenate
TEMPO = (2,2,6,6-Tetramethylpiperidin-1-yl)oxyl
PCC = pyridinium chlorochromate

For a better overview, the synthesis of a particularly preferred compound of formula Id in line with the following example is illustrated in the following scheme 2.

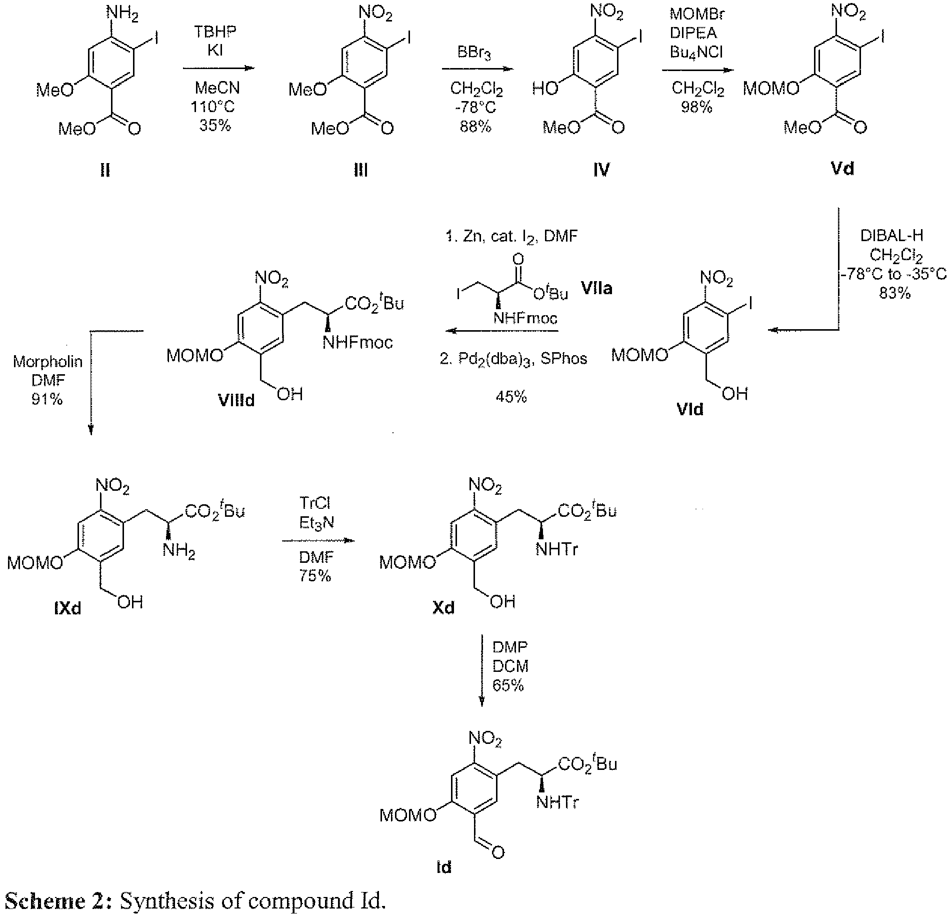

### Synthesis Example 1:

### Synthesis of Methyl 15-iodo-2-methoxy-4-nitrobenzoate III

An autoclave was charged with methyl 4-amino-5-iodo-2-methoxybenzoate II (26 g, 84,7 mmol) and KI (840 mg, 5 mmol) in Acetonitril (180 ml). To the suspension was added *tert-*Butyl hydroperoxide (53,5 mL, 50,3 g, 0,4 mol, 70% in H₂O). The reaction mixture was then stirred at 110°C overnight and dissolved in ethyl acetate (200 mL). The organic phase was washed with an aqueous solution of Na₂S₂O₃, dried over anhydrous Na₂SO₄, filtered and the solvent was removed under reduced pressure. The crude product was purified by flash chromatography by elution with ethyl acetate : petroleum ether 1 : 3 → 100% ethyl acetate to afforded 10 g (35%) of methyl 5-iodo-2-methoxy-4-nitrobenzoate III as a bright yellow solid and 13,2 g of the starting material II. ¹H-NMR (500 MHz, CDCl₃) δ [ppm] 3.93 (s, 3H), 3.95 (s, 3H), 7.45 (s, 1H), 8.34 (s, 1H).

### Synthesis Example 2:

### Synthesis of Methyl 5-iodo-2-hydroxy-4-nitrobenzoate IV

A solution of methyl 5-iodo-2-methoxy-4-nitrobenzoate III (17,8 g, 53 mmol) in methylene chloride (280 mL) was cooled to -78°C an then BBr₃ (15,2 mL, 158 mmol) was added. The mixture was stirred for additional 30 min at -78°C. at which time TLC indicated the reaction to be complete. The reaction was next quenched with ice-cold water and carefully neutralized with a saturated solution of NaHCO₃. The layers were separated and the aqueous phase was extracted two times with methylene chloride (70 mL). The combined organic extracts were dried over anhydrous Na₂SO₄, filtered and concentrated under reduced pressure. The crude residue was purified by column chromatography by elution with ethyl acetate : petroleum ether 1 : 3 to provide 15 g (88%) of methyl 5-iodo-2-hydroxy-4-nitrobenzoate IV as a yellow solid. ¹H-NMR (500 MHz, CDCl₃) δ [ppm] 4.02 (s, 3H), 7.41 (s, 1H), 8.43 (s, 1H), 10.90 (s, 1H).

### Synthesis Example 3:

### Synthesis of Methyl 5-iodo-2-methyloxymethyloxy-4-nitrobenzoate Vd

To a solution of methyl 5-iodo-2-hydroxy-4-nitrobenzoate IV (15 g, 46,5 mmol) in methylene chloride (100 mL) was added tetrabutylammonium chloride (1,7 g, 4,6 mmol) and *N*,*N-*diisopropylethylamine (31,8 mL, 185 mmol) under an inert atmosphere. The red mixture was cooled to 0°C and brommethylmethylether (15,2 mL, 185 mmol) was slowly added over 10 min. When the addition was complete, the cooling bath was removed and the mixture was stirred at ambient temperature overnight. After TLC revealed that the reaction was complete, it was quenched by addition of water (100 mL) followed by extraction three times with methylene chloride (80 mL). The combined extracts were dried over anhydrous Na₂SO₄, filtered and concentrated to give 16,7 g (98%) of methyl 5-iodo-2-methyloxymethyloxy-4-nitrobenzoate Vd, which was used in the next step without further purification. ¹H-NMR (500 MHz, CDCl₃) δ [ppm] 3.51 (s, 3H), 3.92 (s, 3H), 5.28 (s, 2H), 7.69 (s, 1H), 8.32 (s, 1H).

### Synthesis Example 4:

### Synthesis of 5-Iodo-2-methyloxymethyloxy-4-nitrobenzylalcohol VId

To a solution of methyl 5-iodo-2-methyloxymethyloxy-4-nitrobenzoate Vd (16,7 g, 45,5 mmol) in toluene (460 mL) under argon atmosphere at -78°C was added dropwise a solution of diisobutylaluminum hydride in toluene (113 mL, 1.0 M, 113 mmol). The mixture was allowed to reach -35°C over a period of 3 h. After the reaction was complete, as established by TLC analysis, it was quenched by addition of methanol (50 mL) and the cooling bath was removed. An aqueous solution of sodium potassium tartrate was added and the mixture was stirred overnight. The layers were separated and the aqueous layer was extracted two times with ethyl acetate (80 mL). The combined organic phases were dried over anhydrous Na₂SO₄, filtered and concentrated under reduced pressure. The crude residue was purified by column chromatography by elution with ethyl acetate : petroleum ether 1 : 2 to yield 12,8 g (83%) of 5-iodo-2-methyloxymethyloxy-4-nitrobenzylalcohol VId as a yellow solid. ¹H-NMR (500 MHz, CDCl₃) δ [ppm] 2.05 (br s, 1H), 3.48 (s, 3H), 4.73 (s, 2H), 5.26 (s, 2H), 7.66 (s, 1H), 8.04 (s, 1H).

### Synthesis Example 5:

### Synthesis or (S)-tert-Butyl-2-(9H-fluorenylmethoxycarbonylamino)-3-(5-hydroxymethyl-4-methoxymethoxy-2-nitro-phenyl)-propionate VIIId

Zinc dust (5,8 g, 89 mmol) was weighed into a round-bottomed flask with side arm, which was repeatedly evacuated while heating the flask with a heat gun and then flushed with argon. Then dry DMF (41 mL) and iodine (1,25 g, 4,9 mmol) were added. To the resulting suspension N-Fmoc-iodoalanine-*tert-*butyl ester VIIa (14,7 g, 30 mmol) was added immediately, followed by iodine (1,25 g, 4,9 mmol). After a slightly exothermal reaction, the mixture was stirred at room temperature. Thin layer chromatography showed (petroleum ether: ethyl acetate, 5:1) complete consumption of the iodide within 30 min. The reaction mixture was next centrifuged and the colorless supernatant transferred to an argon flushed flask. Pd₂(dba)₃ (1,09 g, 1,2 mmol), SPhos (0,97 g, 2,4 mmol) and 5-iodo-2-methyloxymethyloxy-4-nitrobenzylalcohol VIa (6,7 g, 20 mmol) were added to the solution of the organozinc reagent. The mixture was stirred at ambient temperature overnight and the reaction progress was monitored by TLC. The catalyst was removed by filtration and the mixture was concentrated in vacuo. The crude residue was purified by flash chromatography over silica gel (ethyl acetate : petroleum ether 1 : 5) to afford 5,1 g (45%) of (S)-*tert*-butyl-2-(9*H*-fluorenylmethoxycarbonylamino)-3-(5-hydraxymethyl-4-methoxymethoxy-2-nitrophenyl)-propionate VIIId as a dark brown foam. ¹H-NMR (500 MHz, CDCl₃) δ [ppm] 1.45 (s, 9H), 2.29-2.33 (m, 1H), 3.24 (dd, J = 9.0 Hz, 9.5 Hz, 1H), 3.41 (s, 3H), 3.50 (dd, J = 1H), 4.10-4.12 (m, 1H), 4.19 (dd, J = 8.0 Hz, 1H), 4.25-4.28 (m, 1H), 4.62-4.66 (m, 3H), 5.14-5.20 (m, 2H), 5.57 (d, J = 3.5 Hz, 1H), 7.28-7.31 (m, 2H), 7.38-7.41 (m, 2H), 7.46 (s, 1H), 7.53-7.56 (m, 2H), 7.69 (s, 1H), 7.74 (d, J = 7.5 Hz, 2H).

### Synthesis Example 6:

### Synthesis of (S)-tert-Butyl-2-amino-3-(5-hydroxymethyl-4-methoxymethoxy-2-nitro-phenyl)-propionate XIa

To a solution of (S)-*tert*-butyl-2-(9*H*-fluorenylmethoxycarbonylamino)-3-(5-hydroxymethyl-4-methoxymethoxy-2-nitro-phenyl)-prapionate VIIId (76 mg, 0,13 mmol) in DMF (1,5 mL) was added morpholine (0,4 mL, 4,6 mmol) dropwise and the mixture was stirred at ambient temperature over a period of 1h. After TLC analysis indicated that the reaction was complete the solvent was removed under reduced pressure. The crude residue was purified by column chromatography (ethyl acetate : petroleum ether 2 : 1) to yield 42 mg (91%) of (S)-*tert*-butyl-2-amino-3-(5-hydroxymethyl-4-methoxymethoxy-2-nitro-phenyl)-propionate XId as a brown solid. ¹H-NMR (500 MHz, CDCl₃) δ [ppm] 1.43 (s, 9H), 1.83 (br s, 3H), 3.05 (dd, J = 9.0 Hz, 14.0 Hz, 1H), 3.33 (dd, J = 5.5 Hz, 13.5 Hz, 1H),3.48 (s, 3H), 3.62-3.68 (m, 1H), 4.74 (s, 2H), 5.26 (d. J = 0.5 Hz, 2H), 7.42 (s, 1H), 7.72 (s, 1H).

### Synthesis Example 7:

### Synthesis of (S)-tert-Butyl-2-(tritylamino)-3-(5-hydroxymethyl-4-methoxymethoxy-2-nitrophenyl)-propionate Xa

To a solution of (S)-*tert*-butyl-2-amino-3-(5-hydroxymethyl-4-methoxymethoxy-2-nitrophenyl)-propionate XId (50 mg, 0,14 mmol) in dry DMF (0,5 mL) was added under argon atmosphere trityl chloride (39 mg, 0,14 mmol) and triethyl amine (0,5 mL). The reaction mixture was stirred at ambient temperature over a period of 2h and the reaction progress was monitored by TLC. The solvent was removed under reduced pressure and the resulting residue purified by flash chromatography over silica gel (ethyl acetate : petroleum ether 1 : 5) to afford 63 mg (75%) of (S)-*tert*-Butyl-2-(tritylamino)-3-(5-hydroxymethyl-4-methoxymethoxy-2-nitro-phenyl)-propionate Xd as yellow solid. ¹H-NMR (500 MHz, CDCl₃) δ [ppm] 1.06 (s, 9H), 2.75 (d, J = 10Hz, 1H), 3.20 (dd, J = 8.5 Hz, 13.5 Hz, 1H), 3.30 (dd, J = 5.5 Hz, 13.5 Hz, 1H), 3.50 (s, 3H), 3.59-3.64 (m, 1H), 4.77 (d, J = 4.0 Hz, 1H), 5.30 (dd, 7.0 Hz, 15 Hz, 2H), 7.10-7.18 (m, 9H), 7.29 (d, J = 7.0 Hz, 6H), 7.48 (s, 1H), 7.78 (s, 1H).

### Example 8:

### Synthesis of (S)-tert-butyl-(S)-2-(tritylamino)-3-(5-formyl-4-methoxymethoxy-2-nitro-phenyl)-propionate Id

To a stirred solution of (S)-*tert*-Butyl-2-(tritylamino)-3-(5-hydroxymethyl-4-methoxymethoxy-2-nitro-phenyl)-propionate Xd (1,05 g, 1,8 mmol) in methylene chloride (25 mL) Dess-Martin periodinane (1,12 g, 2,6 mmol) was added at 0°C and the solution was stirred for additional 30 min. before the cooling bath was removed. The mixture was stirred at ambient temperature over a period of 1 h followed by the addition of a saturated solution of NaHCO₃ (25 mL) and Na₂S₂O₃ (1g, 6,3 mmol). It was stirred for 15 min and the aqueous layer was extracted two times with methylene chloride (50 mL). The combined organic extracts were dried over anhydrous Na₂SO₄, filtered and the solvent was removed under reduced pressure. The crude residue was purified by column chromatography to afford 700 mg (65%) of (S)-*tert*-butyl-(S)-2-(tritylamino)-3-(5-formyl-4-methoxymethoxy-2-nitro-phenyl)-propionate Id as a yellowish solid. ¹H-NMR (500 MHz, CDCl₃) δ [ppm] 1.09 (s, 9H), 2,75 (br s, 1H), 3.10 (dd, J = 8.0 Hz, 13.5 Hz, 1H), 3.32 (dd, J = 5.5 Hz, 13.5 Hz, 1H), 3.56 (s, 3H), 3.57-3.63 (m, 1H), 5.37 (d, J = 7.0 Hz, 1H), 5.39 (d, J = 7.0 Hz, 1H), 7.10-7.19 (m, 9H), 7.27-7.30 (m, 6H), 7.82 (s, 1H), 7.91 (s, 1H), 10.5 (s, 1H).

### Example 9:

### Radiosynthesis of [¹⁸F]F-L-DOPA:

The aqueous solution of [¹⁸F]fluoride was collected on a QMA cartridge and the activated [¹⁸F]fluoride was eluted with a solution of TBA (550 µL) into a 10 mL headspacevial. The solvent was azeotropically evaporated under a stream of argon at 100°C by repeated addition of 100 µL acetonitrile. The precusor (S)-*tert*-Butyl-(S)-2-(tritylamino)-3-(5-formyl-4-methoxymethoxy-2-nitro-phenyl)-propionate Id (20 mg, 0,033 mmol) was dissolved in DMSO (1 mL) and the resulting solution was transferred by syringe to the headspacevial. The sealed vial was heated to 110°C for 8 minute and a solution of water/acetonitrile was added. The reaction mixture was passed through a HLB SPE cartridge from Waters and the cartridge was washed with water (20 mL) to remove the phase-transfer catalyst. The cartridge was dried under a powerful stream of argon and the protected and labelled precusor was dissolved in acetonitrile (3 mL). The resulting solution was transferred back to the vial and the solvent was azeotropically evaporated again. The residue was oxidized by the addition of mCPBA (22 mg, 0,13 mmol) in acetronitrile (1 mL). The solution was heated to 75°C over a period of 8 minute while the solvent was slowly removed. Finally the residue was hydrolysed with half concentrated HCl at 50°C and the crude product was purified by semi-preparative HPLC to afford the pure [¹⁸F]F-L-DOPA. The enantiomeric purity of [¹⁸F]F-L-DOPA is >99% and the radiochemical yield is about 20% (+/-5%).

The compounds of formula (I) in accordance with the invention can be advantageously used as precusors for the synthesis of radiotracers such as [¹⁸F]F-L-DOPA. In comparison to the current state of science, the application of these precursors affords higher specific activities. Furthermore, the total reaction time is reduced and the deprotection of the labelled precusor can be carried out under mild reaction conditions. The radiotracer is thus obtained in a high yield and with high chemical and radiochemical purity.

## Claims

1. A compound of formula (I) 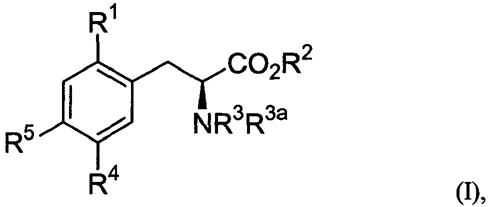 wherein:
R¹ represents a nitro group;
R² represents *tert-*butyl;
R³ and R^{3a} independently represent hydrogen, or a group selected from -CO₂R⁶, -COR⁷ and -R⁸, wherein R⁶ represents C₁-C₆ alkyl which may be substituted, C₂-C₆ alkenyl which may be substituted or aralkyl which may be substituted, R⁷ represents hydrogen or C₁-C₆ alkyl which may be substituted, and R⁸ represents C₁-C₆ alkyl which may be substituted, C₂-C₆ alkenyl which may be substituted, or aralkyl which may be substituted;
R⁴ represents-COR⁹, wherein R⁹ is H;
R⁵ represents -OR¹⁰, wherein R¹⁰ is a linear or branched ether group of the formula - (C₁-C₆ alkyl)-O-(C₁-C₆ alkyl) which can be substituted with a group -O(C₁-C₄ alkyl);
and wherein one or more of hydrogen atoms present in any alkyl or alkylene moiety in the compound of formula I can be substituted by deuterium.

2. The compound of formula (I) as defined in claim 1, which has the following formula (Ic): 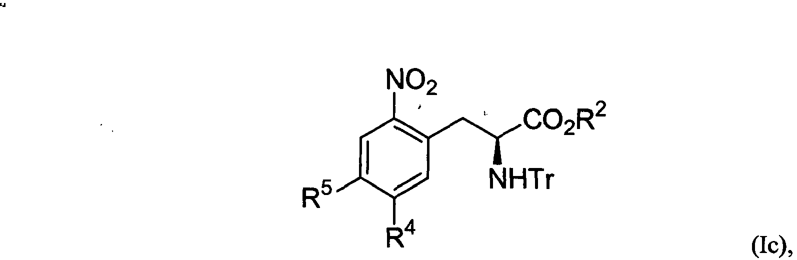 wherein R², R⁴ and R⁵ are as defined in claim 1, and Tr represents a trityl group.

3. The compound of formula (I) as defined in claim 1 or 2, which is the 3-(5-formyl-4-methoxymethoxy-2-nitro-phenyl)-2-(trityl-amino)-propionic acid *tert*-butyl ester of formula (Id): 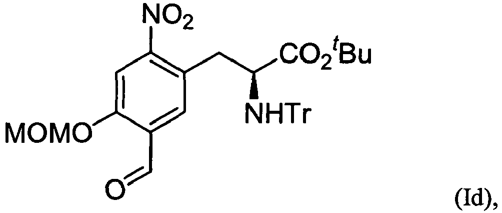 wherein Tr represents the trityl group and MOM represents the methoxymethyl group.

4. Use of a compound of formula (I) as defined in any of claims 1 to 3 as a precursor for the preparation of a ¹⁸F-labelled amino acid.

5. A method for the synthesis of a ¹⁸F-labelled amino acid containing the radiolabel attached to an aromatic ring, said method comprising the steps of
a) providing or obtaining a compound of formula (I) as defined in any of claims 1 to 3, and
b) carrying out a nucleophilic substitution of the leaving group R¹ in the compound of formula (I) by [¹⁸F]fluoride.

6. The method of claim 5, which is a no-carrier-added synthesis.

7. The method of claim 5 or 6, which further comprises, subsequent to step b), a step c1) of converting R⁴ in the compound of formula (I) to a formyl ester.

8. The method of claim 5 or 6, which further comprises, subsequent to step b), a step c2) of replacing the group R⁴ in the compound of formula (I) by a hydrogen atom via decarbonylation.

9. The method of any of claims 5 to 8, which further comprises, subsequent to step b), the step(s) of
d1) removing the group R² or replacing it by hydrogen in order to obtain a free carboxyl group or a salt thereof, and/or
d2) replacing any group(s) R³ or R^{3a} other than hydrogen by hydrogen in order to obtain a free amino group or a salt thereof.

10. The method of any of claims 5 to 9, which method further comprises, subsequent to step b), a step d3) of replacing the group R¹⁰ by hydrogen in order to obtain a free hydroxyl group.

11. The method of any of claims 7, 9 or 10, which comprises the step c1) and which further comprises, subsequent to steps b) and c1), a step d4) of hydrolyzing the formyl ester obtained in step c1) in order to obtain a free hydroxy group.

12. The method of any of claims 7 or 9 to 11 wherein the synthesized ¹⁸F-labelled amino acid is [¹⁸F]Fluor-L-DOPA.

13. The method of any of claims 8 to 10, wherein the synthesized ¹⁸F-labelled amino acid is *para*-[¹⁸F]Fluor-L-tyrosin.

## Patentansprüche

1. Eine Verbindung der Formel (I) 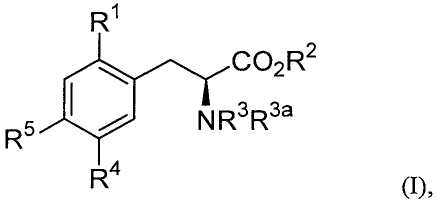 wobei:
R¹ eine Nitrogruppe darstellt;
R² *tert*-Butyl darstellt;
R³ und R^{3a} unabhängig Wasserstoff oder eine Gruppe ausgewählt aus -CO₂R⁶, -COR⁷ und -R⁸ darstellen, wobei R⁶ C₁-C₆-Alkyl, welches substituiert sein kann, C₂-C₆-Alkenyl, welches substituiert sein kann, oder Aralkyl, welches substituiert sein kann, darstellt, R⁷ Wasserstoff oder C₁-C₆-Alkyl, welches substituiert sein kann, darstellt und R⁸ C₁-C₆-Alkyl, welches substituiert sein kann, C₂-C₆-Alkenyl, welches substituiert sein kann, oder Aralkyl, welches substituiert sein kann, darstellt;
R⁴ -COR⁹ darstellt, wobei R⁹ gleich H ist;
R⁵ -OR¹⁰ darstellt, wobei R¹⁰ eine lineare oder verzweigte Ethergruppe der Formel -(C₁-C₆-Alkyl)-O-(C₁-C₆-alkyl) ist, welche mit einer Gruppe -O(C₁-C₄-Alkyl) substituiert sein kann;
und wobei eines oder mehrere Wasserstoffatome in einer beliebigen Alkyl- oder Alkylen-Einheit in der Verbindung der Formel I durch Deuterium ersetzt sein können.

2. Die Verbindung der Formel (I) wie in Anspruch 1 definiert, welche die nachstehende Formel (Ic) aufweist: 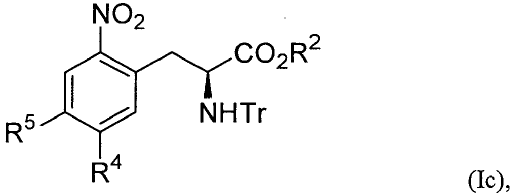 wobei R², R⁴ und R⁵ wie in Anspruch 1 definiert sind und Tr eine Tritylgruppe darstellt.

3. Die Verbindung der Formel (I) wie in Anspruch 1 oder 2 definiert, welche der 3-(5-Formyl-4-methoxymethoxy-2-nitro-phenyl)-2-(trityl-amino)-propionsäure-*tert-*Butylester der Formel (Id) ist: 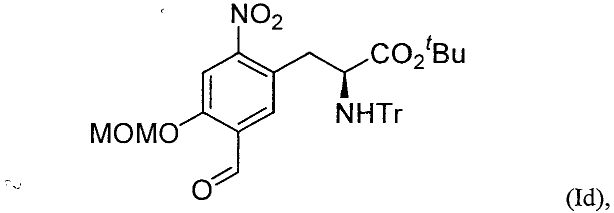 wobei Tr die Tritylgruppe darstellt und MOM die Methoxymethylgruppe darstellt.

4. Verwendung einer Verbindung der Formel (I) wie in einem der Ansprüche 1 bis 3 definiert, als Vorläufer für die Herstellung einer ¹⁸F-markierten Aminosäure.

5. Ein Verfahren zur Synthese einer ¹⁸F-markierten Aminosäure, die die radioaktive Markierung an einen aromatischen Ring gebunden enthält, wobei das Verfahren die nachstehenden Schritte umfasst:
a) Bereitstellen oder Erhalten einer Verbindung der Formel (I) wie in einem der Ansprüche 1 bis 3 definiert, und
b) Durchführen einer nukleophilen Substitution der Abgangsgruppe R¹ in der Verbindung der Formel (I) durch [¹⁸F]Fluorid.

6. Das Verfahren nach Anspruch 5, bei dem es sich um eine "no-carrier added" Synthese handelt.

7. Das Verfahren nach Anspruch 5 oder 6, welches ferner im Anschluss an Schritt b) einen Schritt c1) des Umwandelns von R⁴ in der Verbindung der Formel (I) in einen Formylester umfasst.

8. Das Verfahren nach Anspruch 5 oder 6, welches ferner im Anschluss an Schritt b) einen Schritt c2) des Ersetzens der Gruppe R⁴ in der Verbindung der Formel (I) durch ein Wasserstoffatom mittels Decarbonylierung umfasst.

9. Das Verfahren nach einem der Ansprüche 5 bis 8, welches ferner im Anschluss an Schritt b) den Schritt/die Schritte des
d1) Entfernens der Gruppe R² oder ihres Ersetzens durch Wasserstoff, um eine freie Carboxylgruppe oder ein Salz davon zu erhalten und/oder
d2) Ersetzens jeglicher Gruppe(n) R³ oder R^{3a}, die nicht Wasserstoff sind, durch Wasserstoff, um eine freie Aminogruppe oder ein Salz davon zu erhalten.

10. Das Verfahren nach einem der Ansprüche 5 bis 9, wobei das Verfahren ferner im Anschluss an Schritt b) einen Schritt d3) des Ersetzens der Gruppe R¹⁰ durch Wasserstoff umfasst, um eine freie Hydroxylgruppe zu erhalten.

11. Das Verfahren nach einem der Ansprüche 7, 9 oder 10, welches den Schritt c1) umfasst und welches ferner im Anschluss an die Schritte b) und c1) einen Schritt d4) der Hydrolyse des Formylesters, welcher in Schritt c1) erhalten wurde, umfasst, um eine freie Hydroxygruppe zu erhalten.

12. Das Verfahren nach einem der Ansprüche 7 oder 9 bis 11, wobei die synthetisierte ¹⁸F-markierte Aminosäure [¹⁸F]Fluor-L-DOPA ist.

13. Das Verfahren nach einem der Ansprüche 8 bis 10, wobei die synthetisierte ¹⁸F-markierte Aminosäure *para*-[¹⁸F]Fluor-L-tyrosin ist.

## Revendications

1. Composé de formule (I) 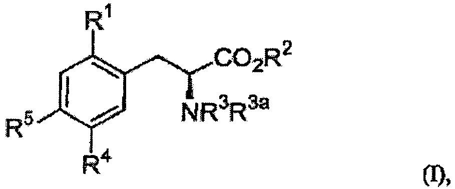 dans lequel :
R¹ représente un groupe nitro ;
R² représente un *tert*-butyle ;
R³ et R^{3a} représentent indépendamment un atome d'hydrogène, ou un groupe choisi parmi -CO₂R⁶, -COR⁷ et -R⁸, où R⁶ représente un alkyle C₁-C₆ qui peut être substitué, un alcényle C₂-C₆ qui peut être substitué ou un aralkyle qui peut être substitué, R⁷ représente un atome d'hydrogène ou un alkyle C₁-C₆ qui peut être substitué, et R⁸ représente un alkyle C₁-C₆ qui peut être substitué, un alcényle C₂-C₆ qui peut être substitué, ou un aralkyle qui peut être substitué ;
R⁴ représente -COR⁹, où R⁹ est H ;
R⁵ représente -OR¹⁰, où R¹⁰ est un groupe éther linéaire ou ramifié de formule -alkyl(C₁-C₆)-O-alkyle (C₁-C₆) qui peut être substitué par un groupe -O-alkyle (C₁-C₄) ;
et dans lequel un ou plusieurs des atomes d'hydrogène présents dans un fragment alkyle ou alkylène quelconque du composé de formule I peuvent être substitués par un deutérium.

2. Composé de formule (I) selon la revendication 1, qui répond à la formule (Ic) suivante : 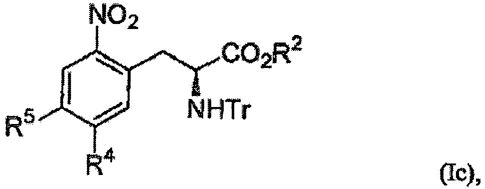 dans lequel R², R⁴ et R⁵ sont tels que définis dans la revendication 1, et Tr représente un groupe trityle.

3. Composé de formule (I) selon la revendication 1 ou 2, qui est l'ester *tert*-butylique de l'acide 3-(5-formyl-4-méthoxyméthoxy-2-nitro-phényl)-2-(trityl-amino)-propionique de formule (Id) : 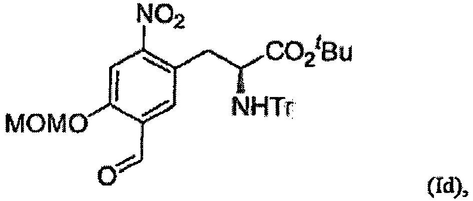 dans lequel Tr représente le groupe trityle et MOM représente le groupe méthoxyméthyle.

4. Utilisation d'un composé de formule (I) selon l'une quelconque des revendications 1 à 3 à titre de précurseur pour la préparation d'un acide aminé marqué par ¹⁸F.

5. Méthode de synthèse d'un acide aminé marqué par ¹⁸F contenant le marqueur radiologique attaché à un cycle aromatique, ladite méthode comprenant les étapes consistant à :
a) fournir ou obtenir un composé de formule (I) selon l'une quelconque des revendications 1 à 3, et
b) effectuer une substitution nucléophile du groupe labile R¹ dans le composé de formule (I) par un fluorure [¹⁸F].

6. Méthode selon la revendication 5, qui est une synthèse sans véhicule ajouté.

7. Méthode selon la revendication 5 ou 6, qui comprend en outre, après l'étape b), une étape c1) qui consiste à convertir R⁴ dans le composé de formule (I) en ester de formyle.

8. Méthode selon la revendication 5 ou 6, qui comprend en outre, après l'étape b), une étape c2) qui consiste à remplacer le groupe R⁴ dans le composé de formule (I) par un atome d'hydrogène par décarbonylation.

9. Méthode selon l'une quelconque des revendications 5 à 8, qui comprend en outre, après l'étape b), les étapes
d1) qui consiste à éliminer le groupe R² ou à le remplacer par un atome d'hydrogène pour obtenir un groupe carboxyle libre ou un sel de celui-ci, et/ou
d2) qui consiste à remplacer tout groupe R³ ou R^{3a} autre que hydrogène par un atome d'hydrogène pour obtenir un groupe amino libre ou un sel de celui-ci.

10. Méthode selon l'une quelconque des revendications 5 à 9, laquelle méthode comprend en outre, après l'étape b), une étape d3) qui consiste à remplacer le groupe R¹⁰ par un atome d'hydrogène pour obtenir un groupe hydroxyle libre.

11. Méthode selon l'une quelconque des revendications 7, 9 ou 10, qui comprend l'étape c1) et qui comprend en outre, après les étapes b) et c1), une étape d4) qui consiste à hydrolyser l'ester de formyle obtenu à l'étape c1) pour obtenir un groupe hydroxy libre.

12. Méthode selon l'une quelconque des revendications 7 ou 9 à 11 dans laquelle l'acide aminé synthétisé marqué par ¹⁸F est une [¹⁸F]Fluor-L-DOPA.

13. Méthode selon l'une quelconque des revendications 8 à 10, dans laquelle l'acide aminé synthétisé marqué par ¹⁸F est une *para-*[¹⁸F]Fluor-L-tyrosine.
